Europäisches Patentamt

European Patent Office

Office européen des brevets

(11) Numéro de publication: **0 524 873 B1**

# (12) FASCICULE DE BREVET EUROPEEN

(45) Date de publication du fascicule du brevet: **11.10.95** (51) Int. Cl.⁶: **A61K 7/48**, A61K 35/78

(21) Numéro de dépôt: **92402100.9**

(22) Date de dépôt: **21.07.92**

Le dossier contient des informations techniques présentées postérieurement au dépôt de la demande et ne figurant pas dans le présent fascicule.

(54) **Préparations cosmétiques antiprurigineuses contenant un extrait de racines d'harpagophyton.**

(30) Priorité: **23.07.91 FR 9109683**

(43) Date de publication de la demande:
**27.01.93 Bulletin 93/04**

(45) Mention de la délivrance du brevet:
**11.10.95 Bulletin 95/41**

(84) Etats contractants désignés:
**AT BE CH DE DK ES FR GB GR IT LI LU MC NL PT SE**

(56) Documents cités:
**EP-A- 0 384 308        FR-A- 2 555 443**
**FR-A- 2 605 224        FR-A- 2 614 791**
**GB-A- 2 103 088        ZA-A- 752 585**

(73) Titulaire: **Sincholle, Daniel**
**343, avenue de la Trémoulette**
**F-34980 Saint-Clement (FR)**

Titulaire: **BONNE, CLAUDE**
**316, avenue d'Occitanie**
**F-34000 Montpellier (FR)**

(72) Inventeur: **Sincholle, Daniel**
**343, avenue de la Trémoulette**
**F-34980 Saint-Clement (FR)**
Inventeur: **BONNE, CLAUDE**
**316, avenue d'Occitanie**
**F-34000 Montpellier (FR)**

(74) Mandataire: **Le Guen, Gérard et al**
**CABINET LAVOIX**
**2, place d'Estienne d'Orves**
**F-75441 Paris Cédex 09 (FR)**

**Description**

La présente invention concerne l'utilisation d'un extrait de racine d' Harpagophyton (*Harpagophytum procumbens D.C., pédaliacées*), (HP) pour la préparation d'une composition permettant de réduire les démangeaisons cutanées. Ces compositions cosmétiques peuvent être pâteuses ou liquides, par exemple des pommades, des crèmes, des gels, des lotions, des compresses imprégnées, et être utilisées pour traiter des démangeaisons de la peau d'origine diverses telles que piqûre d'insecte, prurit d'origine allergique, urticaire, érythème fessier, coup de soleil, etc.

Il est rapporté dans la littérature que HP présente des propriétés thérapeutiques justifiant que cette plante soit souvent utilisée en médecine populaire traditionnelle, pour le traitement des douleurs articulaires mineures.

ZA-A-752 585 concerne des compositions pharmaceutiques à base d'Harpagophyton utilisables dans le traitement de lésions cancéreuses de la peau.

Les inventeurs ont découvert et mis en évidence qu'un extrait de racines de HP présente, indépendamment des propriétés médicinales empiriques, la remarquable propriété d'inhiber la libération de l'histamine, responsable des prurits, à partir de deux types cellulaires, les mastocytes et les basophiles. Ils ont montré en outre, que des préparations cosmétiques contenant un extrait de HP présentent effectivement un effet antiprurigineux.

● Extrait de racines de HP

L'extrait obtenu par infusion dans l'eau bouillante pendant 15 minutes de poudre (granulométrie : 95% $\leq$ 0,8 mm) de racines de HP à raison de 3 g de racines pour 100 ml d'eau. La solution est filtrée en fin de préparation (solution mère). L'extrait de HP est obtenu enfin par évaporation à sec de l'infusé.

● Activité sur les basophiles humains

Un échantillon de sang a été prélevé chez des sujets sélectionnés pour leur sensibilité aux acariens de la poussière de maison.

Après enrichissement en basophiles circulants par sédimentation, les leucocytes ont été préincubés avec des concentrations décroissantes de l'extrait de HP à tester (solution mère diluée au 1/5 à $10^{-7}$) pendant 1/2 heure à la température du laboratoire. Les leucocytes ont été alors incubés à 37 °C en présence de l'allergène sensibilisant. Les basophiles ont été colorés par le Bleu Alcian et comptés au microscope. Les cellules qui n'ont pas réagi, c'est-à-dire qui n'ont pas dégranulé, étaient colorées en rouge du fait de la réaction métachromatique du colorant vis-à-vis des composés héparinoïdes contenus dans les granules.

L'effet protecteur de l'extrait dilué a pu ainsi être évalué. L'histamine libérée par les basophiles dans le surnageant a été dosée par fluorimétrie.

Les résultats de ces expériences sont rapportés dans le tableau 1. On note que les dilutions 1/5 à 1/100 freinent la dégranulation de manière significative et que la dilution 1/5 inhibe significativement la libération d'histamine.

## Tableau 1 : Activité sur les basophiles humains

Dégranulation optique : (% d'inhibition, n = 6)

| Dilutions | 1/5 | 1/10 | $10^{-2}$ | $10^{-3}$ | $10^{-4}$ | $10^{-5}$ | $10^{-6}$ | $10^{-7}$ |
|---|---|---|---|---|---|---|---|---|
| Moyenne | 24,5 | 25,2 | 14,6 | 1,0 | 0,8 | 2,7 | 0 | 5,6 |
| Ecart-type | 11,7 | 14,7 | 8,0 | 6,0 | 6,3 | 9,5 | 7 | 12,7 |
| Analyse statistique | $p<0,05$ | $p<0,05$ | $p<0,05$ | NS | NS | NS | NS | NS |

Libération d'histamine : (% d'inhibition, n = 5)

| | 1/5 | 1/10 | $10^{-2}$ | $10^{-3}$ | $10^{-4}$ | $10^{-5}$ | $10^{-6}$ | $10^{-7}$ |
|---|---|---|---|---|---|---|---|---|
| Moyenne | 19,0 | 19,6 | 2,8 | 12,6 | 2,4 | 6,8 | - 1,0 | 4,0 |
| Ecart-type | 15,1 | 23,4 | 19,5 | 17,2 | 8,4 | 20,0 | 12,0 | 13,1 |
| Analyse statistique | $p <0,05$ | NS | NS | NS | NS | NS | NS | NS |

- Activité sur les mastocytes de rat

Des mastocytes de rat ont été recueillis par lavage péritonéal à l'aide de 10 ml de solution RPMI. Après centrifugation, les mastocytes ont été suspendus dans le RPMI à raison de $10^5$ mastocytes par ml. Les cellules ont été alors mises 14 heures à 37 °C dans un incubateur contenant 5 % de $CO_2$ en présence de dilutions de la solution mère (1/5 - 1/10 - 1/20). L'histamine libérée a été dosée par fluorimétrie . Les résultats sont rapportés dans le tableau 2. Ils montrent une inhibition nette pour les dilutions aux 1/5, 1/10 et 1/20. Seule la dilution au 1/5 atteint le seuil de signification statistique. Aucune dilution de l'extrait testée n'a montré de pouvoir cytotoxique.

## Tableau 2 : Activité sur les mastocytes de rat

Libération d'histamine : (% d'inhibition, n = 4)

| Dilution | 1/5 | 1/10 | 1/20 |
|---|---|---|---|
| Moyenne | 31,0 | 24,7 | 13,5 |
| écart type | 19,2 | 16,9 | 9,5 |
| analyse statistique | $p<0,05$ | NS | NS |

● Activité cutanée chez l'Homme

Une préparation cosmétique contenant 10 % d'extrait sec de HP a été appliquée sur la peau du bras de sujets sains, afin d'évaluer son pouvoir à modifier la réponse cutanée au phosphate de codéine selon la méthode du "prick test".

Les résultats sont rapportés dans le tableau 3. Ils montrent que l'application de la préparation (30 minutes avant le test) est capable de réduire la réaction locale à l'injection de l'agent dégranulant (phosphate de codéine 0,09 %).

## Tableau 3 : Activité cutanée chez l'homme

Aire des papules (mm$^2$)

| Traitement | | | | Excipient | | |
|---|---|---|---|---|---|---|
| Codéine (%) | 9 | 0,9 | 0,09 | 9 | 0,9 | 0,09 |
| Moyenne | 26,8 | 8,8 | 1,1 | 29,9 | 11,5 | 3,7 |
| Ecart type | 2,6 | 1,4 | 0,6 | 2,8 | 1,7 | 1,0 |
| Analyse statistique | NS | NS | p<0,02 | | | |

En conséquence, la présente invention a pour objet l'utilisation d'un extrait de racines d'Harpagophyton pour la préparation d'une composition permettant de réduire les démangeaisons cutanées.

Dans ces compositions pharmaceutiques, l'extrait de racines d'Harpagophyton est généralement présent à une concentration de 1 à 10%.

Ces compositions peuvent en outre contenir éventuellement toutes les substances connues ayant des propriétés bienfaisantes sur les téguments et particulièrement pour leur activité stabilisante antioxydante, ou susceptible d'améliorer la tolérance et/ou la biodisponibilité des principes actifs. Elles peuvent contenir des épaississants, des viscosifiants, des systèmes tampons, des conservateurs et des colorants.

Ces compositions cosmétiques peuvent être présentées sous toutes les formes utilisées en cosmétologie : compresses imprégnées, crème ou gel en pots ou en tubes, lait, lotion en flacon de verre ou de plastique, en ampoules ou en flacons doseurs, etc.

Pour chaque forme particulière, on a recours à des excipients appropriés. A titre d'exemples, on peut citer : le propylèneglycol, la glycérine, les polyols, les phospholipides, mis en liposomes - nanosfères ou microcapsules - ou non, les huiles végétales, animales, minérales, les mouillants, les épaississants, stabilisants et émulsionnants couramment utilisés, l'acide borique et le borax, les phosphates mono- et disodiques, l'édétate disodique, le thimerosal, les parabens, les sels de phénylmercure, les dérivés cellulosiques, les carbopols.

Les différentes formes cosmétiques mentionnées ci-dessus sont obtenues selon les méthodes utilisées dans ce domaine. On donne, ci-après, des exemples de compositions (en parties en poids) :

| 1 - Lotion hydro-alcoolique | |
|---|---|
| **. Extrait sec d'HP** | 10 g |
| . Alcool cétylique | 0,5 |
| . Acide stéarique | 5 |
| . Glycérol | 2 |
| . Alginate de Na | 0,3 |
| .Triéthanolamine | 0,5 |
| . Alcool à 95° | 25 |
| . Eau qsp | 100 |

| 2 - Lait H/E | |
|---|---|
| **. Extrait sec d'HP** | 5,0 g |
| . Polyéthoxyétherphosphate d'alcool oléique | 2 |
| . Alcool cétylique | 0,5 |
| . Lanoline anhydre | 0,5 |
| . Silicone fluide | 1 |
| . Huile de germe de blé | 2 |
| . Huile de cade | 2 |
| . Acide stéarique | 3 |
| . Triéthanolamine | 0,5 |
| . Silicate de Magnésium et d'Aluminium | 0,5 |
| . Composition aromatique | q.s |
| . Conservaturs | q.s |
| . Eau qsp | 100 |

| 3 - Hydrogel alcoolique | |
|---|---|
| **. Extrait sec d'H P** | 3 g |
| . Principes antiseptiques | 0,5 |
| . Triéthylamine | 1,5 |
| . Carbopol 940 | 1,5 |
| . Alcool qsp | 100 |

| 4 - Hydrogel glycériné | |
|---|---|
| **. Extrait sec d'HP** | 1,0 g |
| . Alginate sodique | 8 |
| . Glycérol | 25 |
| . Solution de borax (15 %) dans le glycérol | 20 |
| . Thymol | 2 |
| . Eau qsp | 100 |

EP 0 524 873 B1

| 5 - Crème H/E | |
|---|---|
| . **Extrait sec d'HP** | 1,0 g |
| . Huile de germe de bié | 7 |
| . Stéarate de glycérol | 5 |
| . Propylène glycol | 3 |
| . Triéthanolamine | 0,5 |
| . Lecithine de soja | 1 |
| . Conservateurs | q.s |
| . Eau qsp | 100 |

| 6 - Crème E/H | |
|---|---|
| . **Extrait sec d'HP** | 1,0 g |
| .Acide salicylique | 0,5 |
| . Silicate d'aluminium | 2 |
| . Oxyde de Titane | 2 |
| . Cire blanche | 20 |
| . Huile de paraffine | 60 |
| . Eau qsp | 100 |

**Revendications**

1. Utilisation d'un extrait de racines d'harpagophyton pour la préparation d'une composition thérapeutique permettant de réduire les démangeaisons cutanées.

2. Utilisation cosmétique d'une composition contenant un extrait de racines d'harpagophyton pour réduire les démangeaisons cutanées.

3. Utilisation selon l'une quelconque des revendications 1 à 2, caractérisée en ce que l'extrait d'harpagophyton représente de 1 à 10% en poids de la composition.

4. Utilisation selon la revendication 3, caractérisée en ce que l'extrait est obtenu par infusion des racines dans l'eau bouillante.

5. Utilisation selon l'une quelconque des revendications 1 à 4, caractérisée en ce que la composition est sous forme de lotion, de lait, de gel, de crème, de solution ou de compresse imprégnée.

**Claims**

1. Use of a harpagophyton root extract for preparing a therapeutic composition for reducing skin itching.

2. Cosmetic use of a composition containing a harpagophyton root extract for reducing skin itching.

3. Use according to either one of Claims 1 to 2, characterized in that the harpagophyton extract represents from 1 to 10% by weight of the composition.

4. Use according to Claim 3, characterised in that the extract is obtained by infusing the roots in boiling water.

5. Use according to any one of Claims 1 to 4, characterised in that the composition is in the form of a lotion, milk, gel, cream, solution or impregnated compress.

6

**Patentansprüche**

1. Verwendung eines Harpagophytonwurzelextrakts zur Herstellung einer therapeutischen Zusammensetzung, die die Verminderung von Hautjuckreizen gestattet.

2. Kosmetische Verwendung einer Zusammensatzung, die ein Harpagophytonwurzelextrakt enthält, zur Verminderung von Hautjuckreizen.

3. Verwendung nach einem der Ansprüche 1 bis 2, dadurch gekennzeichnet, daß das Harpagophytonextrakt 1 bis 10 Gew.-% der Zusammensetzung ausmacht.

4. Verwendung noch Anspruch 3, dadurch gekennzeichnet, daß das Extrakt durch Aufgießen der Wurzeln in kochendem Wasser erhalten wird.

5. Verwendung nach einem der Ansprüche 1 bis 4, dadurch gekennzeichnet, daß die Zusammensetzung in Form einer Lotion, einer Milch, eines Gels, einer Creme, einer Lösung oder einer imprägnierten Kompresse vorliegt.